(19) Europäisches Patentamt / European Patent Office / Office européen des brevets

(11) **EP 4 567 394 B1**

(12) **EUROPEAN PATENT SPECIFICATION**

(45) Date of publication and mention
of the grant of the patent:
**10.06.2026 Bulletin 2026/24**

(21) Application number: **24193906.5**

(22) Date of filing: **09.08.2024**

(51) International Patent Classification (IPC):
*G01L 9/00* *(2006.01)*     *G01L 19/00* *(2006.01)*

(52) Cooperative Patent Classification (CPC):
**G01L 9/0042; G01L 9/0054; G01L 19/0092; G01L 19/145**

(54) **PIEZORESISTIVE SENSOR ELEMENT AND PIEZORESISTIVE PRESSURE SENSOR WITH MINIMIZED LONG-TERM AND STERILIZATION DRIFTS**

PIEZORESISTIVES SENSORELEMENT UND PIEZORESISTIVER DRUCKSENSOR MIT MINIMIERTEN LANGZEIT- UND STERILISATIONSDRIFTS

ÉLÉMENT DE CAPTEUR PIÉZORÉSISTIF ET CAPTEUR DE PRESSION PIÉZORÉSISTIF AVEC DÉRIVES À LONG TERME ET DE STÉRILISATION MINIMISÉES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.12.2023 EP 23213911**

(43) Date of publication of application:
**11.06.2025 Bulletin 2025/24**

(73) Proprietor: **TE Connectivity Solutions GmbH**
**8200 Schaffhausen (CH)**

(72) Inventors:
• **KASPAR, Jenni**
**8200 Schaffhausen (CH)**
• **ROMAIN, Jaillet**
**8200 Schaffhausen (CH)**

(74) Representative: **Grünecker Patent- und Rechtsanwälte**
**PartG mbB**
**Leopoldstraße 4**
**80802 München (DE)**

(56) References cited:
**EP-A1- 3 032 235     JP-A- 2018 534 574**
**US-B1- 7 856 885**

**Description**

The present disclosure relates to piezoresistive sensors. In particular, the present disclosure relates to a piezoresistive sensor element and to a piezoresistive pressure sensor that detects mechanical stress acting on a membrane. The present disclosure further relates to sterilizable medical devices, sterile medical devices, and invasive medical devices.

The piezoresistive sensor element according to the present disclosure is based on the physical principle of piezoresistivity, which will be outlined shortly in the following.

Piezoresistive sensors are among the first Micro-Electro-Mechanical-Systems (MEMS) devices and comprise a substantial market share of MEMS sensors in the market today. In particular, silicon piezoresistance has been widely used for various sensors including pressure sensors, accelerometers, cantilever force sensors, inertial sensors, and strain gauges. A detailed overview is for instance given in Barlian, A. Alvin & Park, Woo-Tae & Mallon, Joseph R., Jr. & Rastegar, Ali J. & Pruitt, Beth L. (2009): "Review: Semiconductor Piezoresistance for Microsystems", Proceedings of the IEEE. Institute of Electrical and Electronics Engineers, 97, pp. 513-552, DOI: 10.1109/JPROC.2009.2013612.

It is known that the electrical resistance (R) of a homogeneous material is a function of its dimensions and resistivity ($\rho$),

$$R = \frac{\rho \cdot l}{a} \tag{1}$$

where $l$ is length, and $a$ is average cross-sectional area.

The change in resistance due to applied stress is a function of geometry and resistivity changes. The cross-sectional area of a bulk material reduces in proportion to the longitudinal strain by its Poisson's ratio, $v$, which for most metals ranges from 0.20 to 0.35. For anisotropic silicon, the effective directional Poisson's ratio ranges from 0.06 to 0.36. The isotropic lower and upper limit for $v$ are -1.0 and 0.5. The so-called gauge factor (GF) of a strain gauge is defined as

$$GF = \frac{\Delta R/R}{\varepsilon} \tag{2}$$

where $\varepsilon$ is strain and $\Delta R/R$ is fractional resistance change with strain. The change in resistance is due to both the geometric effects $(1 + 2v)$ and the fractional change in resistivity $(\Delta \rho/\rho)$ of the material with strain

$$\frac{\Delta R}{R} = (1 + 2v)\, \varepsilon + \frac{\Delta \rho}{\rho} \tag{3}$$

Geometric effects alone provide a GF of approximately 1.4 to 2.0, and the change in resistivity, $\Delta \rho/\rho$, for a metal is small-on the order of 0.3. However, for silicon and germanium in certain directions, $\Delta \rho/\rho$ is 50-100 times larger than the geometric term. For a semiconductor, elasticity and piezoresistivity are direction-dependent under specified directions of loads (stress, strain) and fields (potentials, currents). For the sensors according to the present disclosure, the stress induced change of resistivity (the so-called piezoresistive effect) is responsible for the generation of the electrical output signal.

In the following, some basics about the notation and fundamentals of piezoresistivity in semiconductors will be discussed.

As this is generally known, Miller indices can be used for describing crystal structure. Crystals have periodic arrangements of atoms arranged in one of 14 lattice types and complete reviews are available elsewhere. The Miller indices specify crystal planes by n-tuples. A direction index [hkl] denotes a vector normal to a plane described by (hkl), and it represents a family of planes equivalent to (hkl) by symmetry. Angle-bracketed indices, like (hkl), represent all directions equivalent to [hkl] by symmetry. In a hexagonal crystal, as found in most silicon carbide polytypes, the Bravais-Miller index scheme is commonly adopted where four indices are used to represent the intercept-reciprocals corresponding to the four principal crystal axes ($a_1$, $a_2$, $a_3$, and c). The axes $a_1$, $a_2$, and $a_3$ are on the same plane and 120° apart from one another while c is perpendicular to the a-plane defined by the ($a_1$, $a_2$, $a_3$) triplet.

Crystalline silicon forms a covalently bonded diamond-cubic structure with lattice constant a = 5.43 Å. The diamond-cubic structure is equivalent to two interpenetrating face-centered-cubic (FCC) lattices with basis atoms offset by 1/4a in the three orthogonal directions. Silicon's diamond-cubic lattice is relatively sparse (34% packing density) compared to a regular face-centered-cubic (FCC) lattice (74% packing density). Commonly used wafer surface orientations in micromachining include (100), (111), and (110).

Photolithography and etch techniques can create devices in various directions to access desirable material properties. For instance, a (111) oriented piezoresistor in a (110) plane will have the highest piezoresistive sensitivity in a pressure sensor. More commonly, (110) aligned piezoresistors on (100) wafers are used because of their high equal and opposite longitudinal and transverse piezoresistive coefficients.

To define the state of stress for a unit element, nine components, $\sigma_{ij}$, must be specified, as given in the following matrix:

$$\sigma = \begin{bmatrix} \sigma_{11} & \sigma_{12} & \sigma_{13} \\ \sigma_{21} & \sigma_{22} & \sigma_{23} \\ \sigma_{31} & \sigma_{32} & \sigma_{33} \end{bmatrix} \tag{4}$$

The first index i denotes the direction of the applied stress, while j indicates the direction of the force or stress. If $i = j$, the stress is normal to the specified surface, while $i \neq j$ indicates a shear stress on face $i$. From static equilibrium requirements that forces and moments sum to zero, a stress tensor is always symmetric, that is $\sigma_{ij} = \sigma_{ji}$, and thus the stress tensor contains only six independent components.

Strain, $\varepsilon_{ij}$, is also directional. For an isotropic, homogeneous material, stress is related to strain by Hooke's Law, $\sigma = \varepsilon E$. Although "effective" values of Young's modulus and Poisson's ratio for a single direction are often employed for simple loading situations, a tensor is required to fully describe the stiffness of an anisotropic material such as silicon. The stress and strain are related by the elastic stiffness matrix, $C$, where $\sigma_{ij} = C_{ijkl} \varepsilon_{kl}$, or equivalently by the inverse compliance matrix, S, where $\varepsilon_{ij} = S_{ijkl} \sigma_{kl}$:

$$\begin{bmatrix} \sigma_{11} \\ \sigma_{22} \\ \sigma_{33} \\ \sigma_{23} \\ \sigma_{13} \\ \sigma_{12} \end{bmatrix} = \begin{bmatrix} c_{11} & c_{12} & c_{13} & c_{14} & c_{15} & c_{16} \\ c_{21} & c_{22} & c_{23} & c_{24} & c_{25} & c_{26} \\ c_{13} & c_{23} & c_{33} & c_{34} & c_{35} & c_{36} \\ c_{14} & c_{24} & c_{34} & c_{44} & c_{45} & c_{46} \\ c_{15} & c_{25} & c_{35} & c_{45} & c_{55} & c_{56} \\ c_{16} & c_{26} & c_{36} & c_{46} & c_{56} & c_{66} \end{bmatrix} \begin{bmatrix} \varepsilon_{11} \\ \varepsilon_{22} \\ \varepsilon_{33} \\ 2\varepsilon_{23} \\ 2\varepsilon_{13} \\ 2\varepsilon_{12} \end{bmatrix} \tag{5}$$

$$\begin{bmatrix} \varepsilon_{11} \\ \varepsilon_{22} \\ \varepsilon_{33} \\ 2\varepsilon_{23} \\ 2\varepsilon_{13} \\ 2\varepsilon_{12} \end{bmatrix} = \begin{bmatrix} s_{11} & s_{12} & s_{13} & s_{14} & s_{15} & s_{16} \\ s_{21} & s_{22} & s_{23} & s_{24} & s_{25} & s_{26} \\ s_{13} & s_{23} & s_{33} & s_{34} & s_{35} & s_{36} \\ s_{14} & s_{24} & s_{34} & s_{44} & s_{45} & s_{46} \\ s_{15} & s_{25} & s_{35} & s_{45} & s_{55} & s_{56} \\ s_{16} & s_{26} & s_{36} & s_{46} & s_{56} & s_{66} \end{bmatrix} \begin{bmatrix} \sigma_{11} \\ \sigma_{22} \\ \sigma_{33} \\ \sigma_{23} \\ \sigma_{13} \\ \sigma_{12} \end{bmatrix} \tag{6}$$

Collapsed notation reduces each pair of subscripts to one number: $11 \rightarrow 1$, $22 \rightarrow 2$, $33 \rightarrow 3$, $23 \rightarrow 4$, $13 \rightarrow 5$, $12 \rightarrow 6$, e.g., $\sigma_{11}$ to $\sigma_1$, $\varepsilon_{12}$ to $\varepsilon_6$, $c_{1111}$ to $c_{11}$ and $s_{2323}$ to $s_{44}$.

Single crystal germanium and silicon, both of which have a diamond lattice crystal structure, were the first materials widely used as piezoresistors. Piezoresistive coefficients are used for describing the correlation between the electric field components, the current density and the stress. The piezoresistive coefficients ($\pi$) require four subscripts because they relate two second-rank tensors of stress and resistivity. The first subscript refers to the electric field component (measured potential), the second to the current density (current), and the third and fourth to the stress (stress has two directional components). For conciseness, the subscripts of each tensor are also collapsed, e.g., $\pi_{1111} \rightarrow \pi_{11}$, $\pi_{1122} \rightarrow \pi_{12}$, $\pi_{2323} \rightarrow \pi_{44}$. These relations may be generalized for a fixed voltage and current orientation ($\omega$) as a function of stress ($\lambda$):

$$\frac{\Delta \rho_\omega}{\rho} = \sum_{\lambda=1}^{6} \pi_{\omega\lambda} \, \sigma_\lambda$$

$$\tag{7}$$

These coefficients were determined for relatively lightly doped silicon and germanium samples with resistivities ranging from 1.5-22.7 $\Omega$-cm, e.g., 7.8 $\Omega$-cm for p-type silicon. Current commercial and research practice uses doping levels several orders of magnitude higher. Higher concentrations have somewhat lower piezoresistive coefficients, but much lower temperature coefficients of resistance and sensitivity. For example, doping levels are known that result in resistivities in the range of 0.005-0.2 $\Omega$-cm. Piezoresistive coefficients have been measured for (100) samples along the (100) and (110) crystal directions. Longitudinal and transverse coefficients for the fundamental crystal axes were determined directly. Shear piezoresistive coefficients were inferred. By these measurements and considering the crystal symmetry, the piezoresistive tensor of 7.8 Ω-cm silicon was fully characterized according to Smith, C.S. (1954) Piezoresistance Effect in Germanium and Silicon. Physical Review, 94, 42-49, as follows:

$$[\pi_{\omega\lambda}] = \begin{bmatrix} \pi_{11} & \pi_{12} & \pi_{12} & 0 & 0 & 0 \\ \pi_{12} & \pi_{22} & \pi_{12} & 0 & 0 & 0 \\ \pi_{12} & \pi_{12} & \pi_{33} & 0 & 0 & 0 \\ 0 & 0 & 0 & \pi_{44} & 0 & 0 \\ 0 & 0 & 0 & 0 & \pi_{44} & 0 \\ 0 & 0 & 0 & 0 & 0 & \pi_{44} \end{bmatrix} =$$

$$\begin{bmatrix} 6.6 & -1.1 & -1.1 & 0 & 0 & 0 \\ -1.1 & 6.6 & -1.1 & 0 & 0 & 0 \\ -1.1 & -1.1 & 6.6 & 0 & 0 & 0 \\ 0 & 0 & 0 & 138.1 & 0 & 0 \\ 0 & 0 & 0 & 0 & 138.1 & 0 \\ 0 & 0 & 0 & 0 & 0 & 138.1 \end{bmatrix} \times 10^{-11} Pa^{-1}$$

(8)

For a p-type piezoresistor implanted on (100) silicon along the (100) crystal direction, the transversal and longitudinal piezoresistive coefficients may be defined and approximated as follows:

$$\pi_t = \frac{1}{2}(\pi_{11} + \pi_{12} - \pi_{44}) = -\frac{\pi_{44}}{2} \tag{9}$$

$$\pi_l = \frac{1}{2}(\pi_{11} + \pi_{12} + \pi_{44}) = \frac{\pi_{44}}{2} \tag{10}$$

Thus, the resulting change in resistance is

$$\frac{\Delta R}{R} = \sigma_l \pi_l + \sigma_t \pi_t \tag{11}$$

$$\frac{\Delta R}{R} = \pi_{44}(\sigma_l - \sigma_t) \tag{12}$$

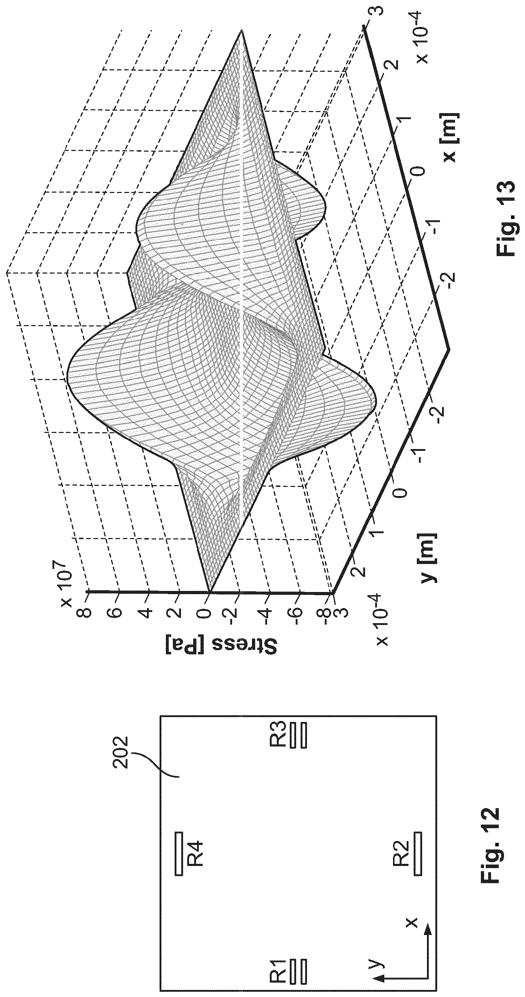

Fig. 13 illustrates a simulation of the stress values $\sigma_l$ - $\sigma_t$ for a membrane 202 as depicted in Fig. 12 when a pressure of 1 bar is applied from the back. In this example, the membrane has dimensions of 390 μm in the x-direction, 390 μm in the y-direction, and a thickness of 6 μm.

It should be noted that a difference has to be made between the terms stress and strain.

When a material is put under pressure or has a mechanical load applied to it, mechanical stress is developed. When a solid is put under stress, it has the ability to deform. This deformation is called strain. The stress is the pressure per unit area of the material, and the resulting strain is the deformation that occurs as a result of this stress. Strain and stress are strongly intertwined because strain occurs solely as a result of stress. Stress is defined as the force per unit area generated within materials as a result of externally applied forces, unequal heating, or persistent deformation. The unit for stress is Nm$^{-2}$ (Pa). On the other hand, strain is defined as the amount of distortion experienced by the body in the direction of force compared to the original dimension of the object. Strain defines the relative change in the shape of an object.

The piezoresistors used for the piezoresistive sensor elements according to the present disclosure are responsive to the stress they experience.

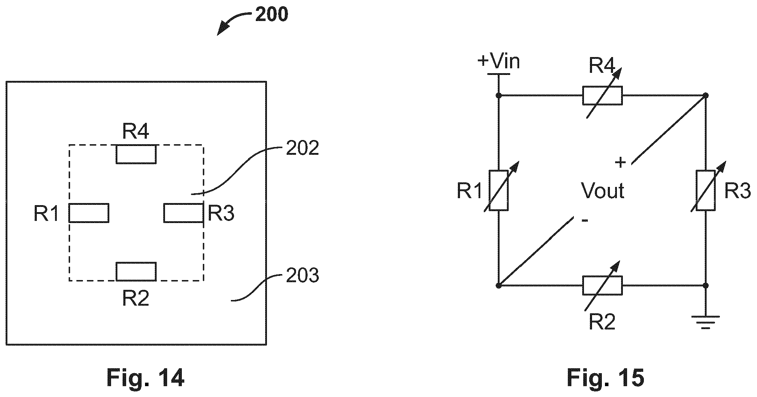

Furthermore, it is known in the art to use the concept of a Wheatstone bridge in order to ensure a precise and offset-free measurement. Fig. 14 illustrates a schematic representation of a MEMS pressure sensor 200 comprising four stress sensitive piezoresistors R1, R2, R3, R4. A deflectable membrane (also referred to as diaphragm) 202 is surrounded by a stiffer frame 204. Pressure that acts on the membrane 202 causes stress in the membrane which is sensed by the four stress sensitive piezoresistors R1, R2, R3, R4.

These piezoresistors are arranged in the Wheatstone bridge circuit as depicted in Fig. 15 for a full-bridge configuration. When no pressure is applied, the output voltage, Vout is 0 V as all the piezoresistors are equal to $R_0$ *(1+α∆T)*. When the pressure is applied, the diaphragm is under stress. The active piezoresistors, which are positioned in the high stress regions on the diaphragm are strained, hence change their values to $R_0$ (1+$α∆T$ +$π_l$ $∆σ_l$ +$π_t$ $∆σ_t$) where $α$ is the temperature coefficient of resistance. $π_l$ and $π_t$ are the longitudinal and transverse piezoresistive coefficients, respectively. Meanwhile $∆T$, $∆σ_l$, and $∆σ_t$ are the changes in temperature, longitudinal stress and transverse stress, respectively. For example with pressure coming from the top of the membrane, the active perpendicular resistors (R1 and R3) experience mostly the longitudinal stress and the active parallel resistors (R2 and R4) undergo mostly the transverse stress. In other words, the resistors all experience both stresses. The sum of these two stresses is drawn in Fig. 13, illustrating why two of these resistors increase in value while the two others decrease when the membrane is exposed to pressure. A longitudinal tensile stress increases the resistivity of p-type resistors, while a transverse stress has the opposite effect. Hence, the Vout changes accordingly due to these changes. Assume that ∆R1=∆R3 and ∆R2=∆R4, thus, the Vout expressions for the full-bridge (Fig. 15) are given by the following expression:

$$Vout = Vin \left( \frac{\pi_l \Delta \sigma_l - \pi_t \Delta \sigma_t}{2(1+\alpha \Delta T) + \pi_l \sigma_l - \pi_t \Delta \sigma_t} \right) \tag{13}$$

As this is generally known, many disturbances that act uniformly on all four resistors can be eliminated by using the Wheatstone bridge configuration. Examples of such disturbances include process variations such as an implant dose, a linewidth and changes of the resistors due to temperature.

However, it has been found that conventional piezoresistive sensors suffer from slow long-term drift effects that are for instance caused by electrostatic build-up (for instance in a covering gel or a plastic cap) and/or environmental effects and/or process issues such as ionic contamination, material impurities, electromigration, stress release, stress in material layers, process residual stress, thermal coefficient (TC) mismatch, TC change etc. Stress isolation may cause such a drift, but this effect is not considered for the present disclosure because there exist concepts to limit this drift effect.

Moreover, conventional piezoresistive sensors suffer from initial drift effects caused by sterilization processes. When sterilization is performed, e.g., by irradiating the calibrated sensor with ionizing radiation, drift in pressure measurement occurs due to the creation of defects in the semiconductor material, charge trapping, material degradation, and changes in doping levels. These effects alter the electrical properties and resistance of the sterilized sensor, thereby affecting its functionality.

A slow drift of the offset caused by external disturbances cannot be discerned from slow changes of the measurand. Furthermore, an initial drift due to sterilization of a calibrated sensor leads to incorrect measurements for the whole device life. In particular for the application of a MEMS pressure sensor, these drawbacks lead to a functional safety concern. In particular, relevant standards in the application fields of automotive industry, or safety in medical applications such as for respiratory devices. In the context of the present disclosure, the term long-term drift relates to time spans between several months and about two years, preferably about one year, while the term initial drift refers to an effect that occurs almost instantly after sterilization.

Some conventional sensor concepts deal with the long-term drift problem using a buried field shield for compensating the effect of any ionic contaminations due to the fabrication process. Further, it is known to use a polycrystalline silicon or metal field shield to nullify external electric fields. Some conventional sensors comprise a sensor housing having metal caps with lid shields in order to avoid electrostatic charge build-up.

Furthermore, some conventional sensor concepts deal with the initial drift problem using a radiation shield to protect the electronic circuitry, including the piezoresistors, from the ionizing radiation. For example, the radiation shield can be a coating of one of more layers of materials that scatter the ionizing particles to reduce their impact energy.

However, these counter-measures complicate the manufacturing process and often do not achieve satisfactory results.

Moreover, EP 3287758 B1 discloses a differential pressure sensor, which may provide a common mode corrected differential pressure reading. The differential pressure sensor includes for instance two pressure sensing diaphragms. The pressure sensor may be configured so that the first diaphragm measures the differential pressure between two sections of a fluid. The pressure sensor may also be configured so that the second diaphragm measures the common mode error experienced by the die at the time the differential pressure is read by the first diaphragm. Electrical connectors may be configured so that the differential pressure outputs a common mode error corrected differential pressure reading based on the readings of the first and second diaphragm. In particular, the second diaphragm includes at least one pressure sensitive electrical element that exhibits a varying resistance responsive to deflection of the diaphragm of the second pressure sensing die that is representative of a common mode error of the second pressure sensing die.

European patent application 22198016.2 relates to another differential pressure sensor. According to this document, the drift signal of the drift pressure sensor (i.e. a signal generated by a drift sensing unit formed on or in the symmetrical diaphragm) can additionally be used to trigger a warning signal. In more detail, by comparing the drift signal with a predefined threshold value, it is possible to estimate whether the differential pressure sensor (in particular a signal generated by a differential sensing unit formed on or the differential diaphragm) has been degraded.

This enables the technical effect that the differential pressure sensor can have a longer lifetime, in particular the pressure sensor does not need to be replaced after a predefined fixed time but can be replaced on demand (namely when the warning signal, which is sensed by the drift pressure sensor, is output).

US7856885 B1 discloses a piezo-resistive pressure sensor array comprising of a double diaphragm, with the outer diaphragm acting both as reinforcement and sensing structure along with an inner sensing diaphragm and a temperature compensation bridge.

However, there is still the need for a piezoresistive sensor, which allows a particularly precise and long-term stable measurement, at the same time being fabricated in a cost efficient manner.

This object is solved by the subject matter of the independent claims.

Advantageous embodiments of the present disclosure are the subject matter of the dependent claims.

The present disclosure is based on the idea to provide a second Wheatstone bridge in addition to the main bridge for monitoring and compensating environmental and/or process induced long-term and initial drift effects. In particular, a piezoresistive sensor element according to the present disclosure comprises a substrate, which in operation is subjected to mechanical stress in response to a measurand to be measured, and a first array of at least four sensitive piezoresistors, wherein the sensitive piezoresistors are arranged on the substrate and are connected to form a first Wheatstone bridge for generating a first bridge signal. Further, a second array of at least four in-sensitive piezoresistors is provided, wherein the in-sensitive piezoresistors are connected to form a second Wheatstone bridge for generating a second bridge signal, and wherein the sensitive piezoresistors have a stress sensitivity which is higher than the stress sensitivity of the in-sensitive piezoresistors, and wherein an output signal of the piezoresistive sensor is generated based on a difference of the first and second bridge signals.

This solution has the advantage that the long-term and initial drifts can compensated by generating an output signal based on a difference, i.e. by comparing the first and second bridge signals. One may subtract the bridge signals, and this subtraction can be performed downstream, for example by a signal processing unit, or directly by physical connecting the outputs of the two Wheatstone bridges, that is, by cross-coupling the two Wheatstone bridges, while in the latter case, the sensitivity of the main bridge is reduced, whereas in the former case, the sensitivity of the main bridge remains unaffected. In all cases, the reliability and safety of the sensor is enhanced.

According to an advantageous example of the piezoresistive sensor element, the substrate comprises silicon, wherein each of the piezoresistors comprises doped areas ion-implanted in the silicon material. Silicon is a well-established material in the semiconductor industry and offers the potential for using established CMOS processes for fabricating the sensor element and further electronic components. As mentioned in the theoretical part above, monocrystalline silicon also exhibits a strong piezoresistive effect, in particular when using ion implantation for fabricating the piezoresistors.

However, it is clear that other materials showing a piezoresistive effect (for instance germanium or silicon carbide) may also be used in connection with the present disclosure. Further, for the fabrication of the piezoresistors, also other known technologies such as diffusion, epitaxy, or deposition of a doped polycrystalline silicon layer can also be employed.

When manufacturing the piezoresistive sensor element using a monocrystalline material, such as monocrystalline silicon, the piezoresistivity may be anisotropic. Advantageously, each of the sensitive piezoresistors may be oriented along a first crystallographic orientation of the substrate and each of the corresponding in-sensitive piezoresistors may be oriented along a second crystallographic orientation of the substrate, the first crystallographic orientation being different from the second crystallographic orientation and causing a higher stress sensitivity than the second crystallographic orientation.

For instance, the substrate may be fabricated from p-type silicon with a (100) plane forming the outer surface, wherein the sensitive piezoresistors are arranged along a [$\overline{1}10$] direction and/or a [110] direction, and wherein the in-sensitive piezoresistors are arranged along a [100] direction and/or a [010] direction. A particularly high sensitivity and accuracy can be achieved with this configuration.

Alternatively, the substrate may also be fabricated from n-type silicon with a (100) plane forming the outer surface, wherein the sensitive piezoresistors are arranged along a [100] direction and/or a [010] direction and/or a [010] direction, and wherein the in-sensitive piezoresistors are arranged along a [110] direction and/or a [110] direction.

In order to ensure that the elements of the second Wheatstone bridge experience the same disturbances as the piezoresistors of the first Wheatstone bridge, so that all long-term drift effects match as closely as possible, each of the in-sensitive piezoresistors may be arranged in close proximity to one corresponding sensitive piezoresistor, so as to be subjected to essentially the same stress as the corresponding sensitive piezoresistor.

According to claim 1, the in-sensitive piezoresistors are arranged to include an angle of 45 degrees with each of the sensitive piezoresistors.

According to a further advantageous example, the piezoresistive sensor element further comprises a signal processing unit for evaluating the first bridge signal and the second bridge signal and for generating the sensor output signal. Thus, no external signal processing units and connection leads have to be involved for the compensation calculus, so that the accuracy and reliability can be increased. The signal processing unit may be or comprise an application-specific integrated circuit, henceforth shortened as ASIC. The ASIC may be encrypted, so that the sensor output signal may be encrypted.

The most accurate and compact architecture can be achieved when the signal processing unit is monolithically integrated with the piezoresistors on the same chip (also called die).

The effect of temperature influences may be monitored either be using the inherent temperature coefficient of resistance (TCR) of the second Wheatstone bridge or by additionally providing a temperature sensor, such as a temperature diode, arranged within the second Wheatstone bridge. Such a temperature diode is also known as a thermal diode. The functioning of a thermal diode is based on the property of electrical diodes to change voltage across it linearly according to temperature.

In another example, the signal processing unit may comprise a first signal processing subunit for evaluating the first bridge signal and generating a first preliminary signal and a second signal processing subunit for evaluating the second bridge signal and generating a second preliminary signal. Each of the signal processing subunits may be or comprise an ASIC for generating the respective preliminary signal.

The piezoresistive sensor element may further comprise a microcontroller connected to the signal processing subunits for computing the sensor output signal based on a difference between the first preliminary signal and the second preliminary signal.

The signal processing unit may compensate for contributions to the first and second bridge signals that are not due to mechanical stress, e.g. a long-term drift and/or an initial drift due to ionizing radiation.

According to claim 1, the first Wheatstone bridge are cross-coupled to the second Wheatstone bridge. The cross-coupling of the first and second Wheatstone bridges automatically compensates for contributions to the first and second bridge signals that are not due to mechanical stress.

In another example, the piezoresistive sensor element may further comprise a status indicator configured to provide a first flag output before sterilization of the piezoresistive sensor element and a second flag output after sterilization of the piezoresistive sensor element. The sterilization may be performed with a predetermined medically sterilizing dose. With a "medically sterilizing dose", it is meant that the dose is above its minimum required for medical sterilization of the piezoresistive sensor element.

The status indicator may be configured to provide the second flag output if a deviation of the first bridge signal from the second bridge signal exceeds a predetermined medically sterilizing signal threshold and to provide the first flag output if a deviation of the first bridge signal from the second bridge signal does not exceed the predetermined medically sterilizing signal threshold. The predetermined medically sterilizing signal threshold is representative of the predetermined medically sterilizing radiation dose.

The status indicator may be further configured to provide a third flag output when an ionizing radiation dose received by the piezoresistive sensor element exceeds a predetermined damage dose. With a "damage dose", it is meant that the dose is above its minimum that negatively affects the functionality of the piezoresistive sensor element. The status indicator may be configured to provide the third flag output when the deviation of the first bridge signal from the second bridge signal exceeds a predetermined damage signal threshold that is representative of the predetermined damage dose.

The status indicator may be accessible from outside and comprise a terminal or generate a digital signal.

As mentioned above, the architecture proposed in the present disclosure is intended to eliminate the detrimental effects of long-term drift effects due to electrostatic build-up or external or process induced ionic influences and initial drift effects due to sterilization processes. In order to enhance the effect of these influences on the second Wheatstone bridge, the passivation layer, which is present on the piezoresistive sensor element, may at least partly removed in regions above the in-sensitive piezoresistors.

The present disclosure relates exemplarily to a piezoresistive pressure sensor comprising at least one piezoresistive sensor element according to the principles described above, wherein the substrate comprises a deflectable membrane (which may also be referred to as a diaphragm), which in operation is deflected in response to a pressure to be measured.

It is clear that the ideas of the present disclosure may also be applicable to other piezoresistive sensors, such as force and inertial sensors. For instance, cantilever sensors, strain gauges, accelerometers, and gyroscopes may be equipped with piezoresistive sensor elements in line with the present disclosure. However, piezoresistive pressure sensors are some of the most reported and developed micromachined devices. Thus, the following detailed description will focus on piezoresistive pressure sensors, which typically measure deformation of a thin circular or rectangular membrane (diaphragm) under an applied external pressure. The membrane may be made from the same material as the wafer substrate (silicon, diamond, etc.) or CVD-based thin films (oxide, nitride, etc.). Integrated piezoresistors are formed by dopant diffusion, ion implantation, or doped epitaxy. However, it is clear that other materials showing a piezoresistive effect (for instance germanium or silicon carbide) may also be used in connection with the present disclosure.

According to an advantageous example, the membrane is surrounded by a frame having a higher stiffness than the membrane, and wherein the in-sensitive piezoresistors are arranged on the frame. Thus, the in-sensitive piezoresistors are decoupled from deformation of the membrane. The in-sensitive piezoresistors may in this case be arranged close to the periphery of the diaphragm or further away from the diaphragm. However, the in-sensitive piezoresistors are arranged on the deflectable membrane itself, so as to experience exactly the same stress and environment as the sensitive piezoresistors.

According to an advantageous example, the membrane has a rectangular outline and the sensitive piezoresistors have an elongated shape, and wherein a first pair of the sensitive piezoresistors are arranged along opposing sides of the deflectable membrane's outline and a second pair of the sensitive piezoresistors are arranged to include an angle with the other opposing sides of the deflectable membrane's outline.

According to another aspect of the invention, there is provided a sterilizable medical device for pressure measurements comprising a piezoresistive sensor element and being configured to be sterilized by ionizing radiation and/or ethylene oxide as defined in claim 9. The signal processing unit may be configured to use the second bridge signal for generating a flag signal that indicates whether the piezoresistive sensor element has been sterilized.

When the sterilizable medical device is used, e.g. is implanted or is in fluid connection with body fluids, it has to be sterile, i.e. it has to be free from all biological contaminants. Possible sterilization methods use ionizing radiation and/or ethylene oxide. When the sterilization is performed by ionizing radiation, a typical dose is around 60 kGy. When the sterilization occurs by an electron beam, the energy of the electrons can be of over 1 MeV. The sterilization dose may be about 30kGy, applied with about 9.8 meV as beam energy.

Therefore, according to another aspect of the invention, there is provided a sterile medical device for pressure measurements comprising the sterilizable medical device, wherein the sterilizable medical device has been sterilized, e.g., by a predetermined dose of ionizing radiation or by treatment with ethylene oxide as defined in claim 10.

The sterile medical device for pressure measurements may further comprise an interface configured for sterile coupling to a patient. Moreover, the sterile medical device may be configured to be in contact with a body fluid of the patient, typically blood. The contact may be realized through an intravenous tube, possibly connected to an artery when the device measures arterial blood pressure, or a needle for subcutaneous delivery of a medicine.

The sterile medical device may be used for invasive blood pressure measurements. According to yet another aspect of the invention, there is provided an invasive medical device configured to be sterilized by ionizing radiation and/or ethylene oxide and comprising a piezoresistive pressure sensor. The invasive medical device may be configured to contact a body fluid of a patient, and the piezoresistive pressure sensor may be configured to measure a pressure of said body fluid. The accompanying drawings are incorporated into the specification and form a part of the specification to illustrate several embodiments of the present disclosure. These drawings, together with the description serve to explain the principles of the disclosure. The drawings are merely for the purpose of illustrating the preferred and alternative examples of how the disclosure can be made and used, and are not to be construed as limiting the disclosure to only the illustrated and described embodiments. Furthermore, several aspects of the embodiments may form-individually or in different combinations-solutions according to the present disclosure. The following described embodiments thus can be considered either alone or in an arbitrary combination thereof. Further features and advantages will become apparent from the following more particular description of the various embodiments of the disclosure, as illustrated in the accompanying drawings, in which like references refer to like elements, and wherein:

FIG. 1    is a schematic top view of a piezoresistive pressure sensor according to a first example;

FIG. 2    is a schematic is a schematic cross sectional view of the piezoresistive pressure sensor shown in Fig. 1;

FIG. 3    is a schematic representation illustrating the crystallographic orientation of the piezoresistors according to an advantageous example;

FIG. 4    is a schematic representation of a piezoresistive pressure sensor according to a further example;

FIG. 5    is a schematic representation of the electronic signal processing according to an advantageous example;

FIG. 6    is a schematic representation of a piezoresistive pressure sensor according to a further example;

FIG. 7    is a schematic representation of a piezoresistive pressure sensor according to a further example;

FIG. 8    is a schematic top view of a piezoresistive pressure sensor according to a further example;

FIG. 9    is a schematic is a schematic cross sectional view of a piezoresistive pressure sensor according to a further example;

FIG. 10   is a schematic top view of a piezoresistive pressure sensor according to a further example;

FIG. 11   is a schematic representation of the electronic signal processing according to a further advantageous example;

FIG. 12   is a schematic top view of a diaphragm region of a conventional piezoresistive pressure sensor;

FIG. 13   is schematic illustration of the stress distribution within the piezoresistive pressure sensor of Fig. 12;

FIG. 14   is a schematic top view of a conventional piezoresistive pressure sensor;

**FIG. 15** is a circuit diagram of a Wheatstone bridge comprising the piezoresistors shown in Fig. 14; and

**Fig. 16** a schematic representation of a piezoresistive pressure sensor with cross-coupling in a further example.

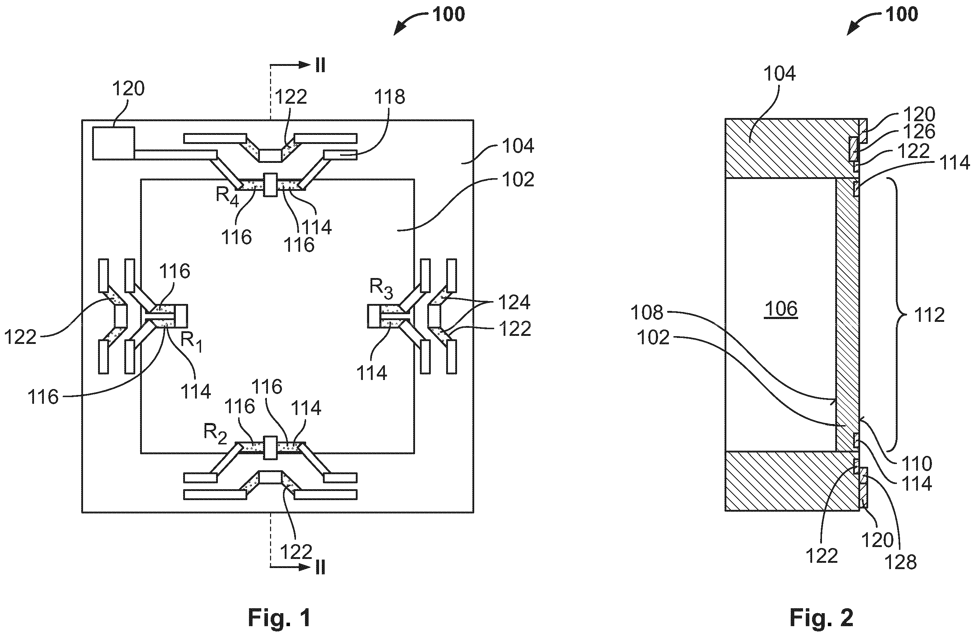

The present disclosure will now be explained in more detail with reference to the Figures and firstly referring to Fig. 1 and 2.

Fig. 1 is a schematic top view of a piezoresistive pressure sensor 100 according to a first example of the present disclosure. Fig. 2 is the corresponding sectional view along the cut line II-II of Fig. 1.

According to this particular example, the piezoresistive pressure sensor 100 has a rectangular outline. Of course, the principles according to the present disclosure may also be applied to sensor structures with a different outline, e.g. a circular outline. The piezoresistive pressure sensor 100 comprises a deflectable membrane 102 which is also referred to as a diaphragm. The membrane 102 is surrounded and supported by a thicker and thus stiffer frame 104.

The membrane 102 and the frame 104 define a void 106, in which the pressure to be measured is allowed to act on the first (inner) side 108 of the membrane. The second side 110 of the membrane is oriented outwardly.

When a pressure difference is built up between the first side 108 and the second side 110 of the membrane 102, mechanical stress is generated. The membrane is deflectable and the maximal stress occurs on the edge. This may for instance be seen from Fig. 13. The maximum stress is therefore generated in the area 112 defined by the membrane 102. The frame 104 is the location where the stress caused by the pressure is minimal.

According to the present disclosure, a group of four sensitive piezoresistors 114 is arranged on the membrane 102 around the perimeter of the membrane 102. According to the shown example, each of the sensitive piezoresistors 114 comprises one or more, for instance two, ion implanted resistive regions 116 and electrically conductive connecting leads 118, which interconnect the resistive regions 116 and allow the interconnection between the sensitive piezoresistors 114 and to a signal processing circuit. Bond pads 120 may be provided for the electrical connection of the die for instance by wire bonding, solder bumps in flip-chip technology, or the like. In Figs. 1 and 2, only one of the pads 120 is shown in order to keep the schematic drawing simple.

The sensitive piezoresistors 114 in operation of the pressure sensor 100 change their resistivity in response to mechanical stress in the membrane with the maximum possible sensitivity. The four piezoresistors 114 are interconnected with each other to form a first Wheatstone bridge 130 as explained above and shown in Fig. 15.

According to the present disclosure, in addition to these sensitive piezoresistors 114, a group of in-sensitive piezoresistors 122 is provided for compensating the effects of long-term drift. The insensitive piezoresistors 122 react towards mechanical stress with no or only a very low sensitivity. Thus, these in-sensitive piezoresistors 122 are essentially only perceptive of the drift causing influences. The in-sensitive piezoresistors 122 are connected to form a second Wheatstone bridge 132 and the sensor output signal can be based on a difference, i.e. comparing the first bridge signal and the second bridge signal in order to yield an accurate and drift-compensated sensor signal. For example, the second bridge signal can be subtracted from the first bridge signal. The subtraction can be performed downstream, for example by a signal processing circuit, or, as required by claim 1, directly by physical connecting the outputs of the two Wheatstone bridges 130, 132 by cross-coupling the two bridges as explained in more detail below.

The in-sensitive piezoresistors 122 may each comprise one or more interconnected ion implanted resistive regions 124.

As schematically illustrated in Fig. 2, the connecting leads 118 may for instance comprise diffused conductors 126 or deposited conductive layers, e. g. metal layers 128.

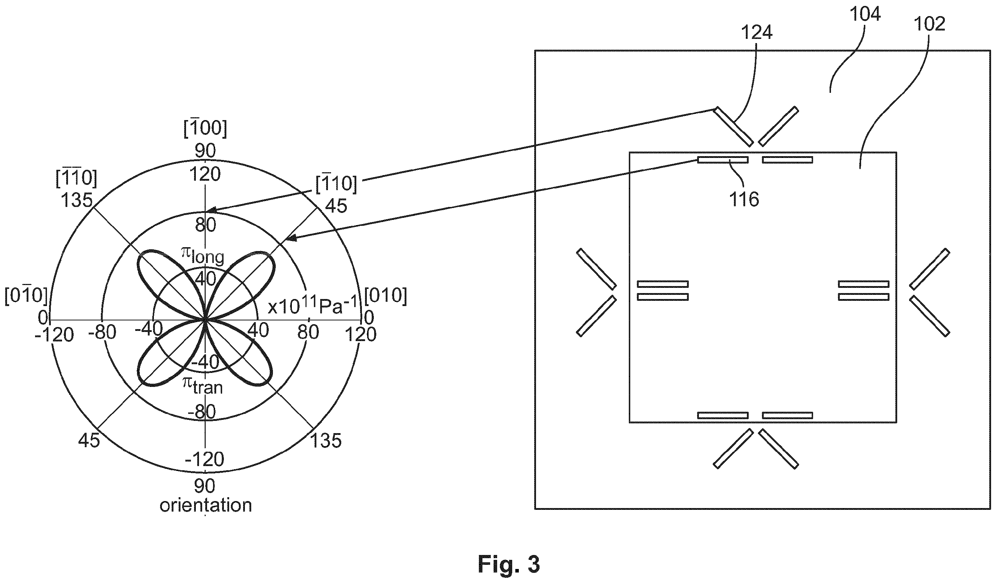

The example shown in Figs. 1 and 2 shows a piezoresistive pressure sensor 100, which is based on p-type monocrystalline silicon. As can be derived from Fig. 1, the resistive regions 124 of the stress in-sensitive piezoresistors 122 are laid out at an angle of 45 degrees compared to the resistive regions 116 of the stress sensitive piezoresistors 114. The reason for this arrangement will now be explained with reference to Fig. 3.

In particular, in Fig. 3 the orientation of the ion implanted resistive regions 116 of the stress sensitive piezoresistors 114 and the ion implanted resistive regions 124 of the stress in-sensitive piezoresistors 122 is compared to the piezoresistive coefficients in the (100) plane of a p-type silicon substrate as for instance shown in Barlian, A. Alvin & Park, Woo-Tae & Mallon, Joseph R., Jr. & Rastegar, Ali J. & Pruitt, Beth L. (2009): "Review: Semiconductor Piezoresistance for Microsystems", Proceedings of the IEEE. Institute of Electrical and Electronics Engineers, 97, pp. 513-552, DOI: 10.1109/JPROC.2009.2013612.

The ion implanted resistive regions 116 of the stress sensitive piezoresistors 114 are arranged along the [110] direction and the [110] direction, so that these piezoresistors exhibit the maximum possible stress sensitivity. In contrast thereto, the ion implanted resistive regions 124 of the stress in-sensitive piezoresistors 122 are arranged along the [100] direction and the [010] direction, thus exhibiting no or only a negligible stress sensitivity.

Although not shown in the Figures, it should be noted that for n-type silicon the sensitive piezoresistors have to be arranged along the [100] direction, the [010] direction and/or the [010] direction, whereas the in-sensitive piezoresistors are arranged along the [$\overline{1}$10] direction and/or the [110] direction, in order to achieve an analogous result.

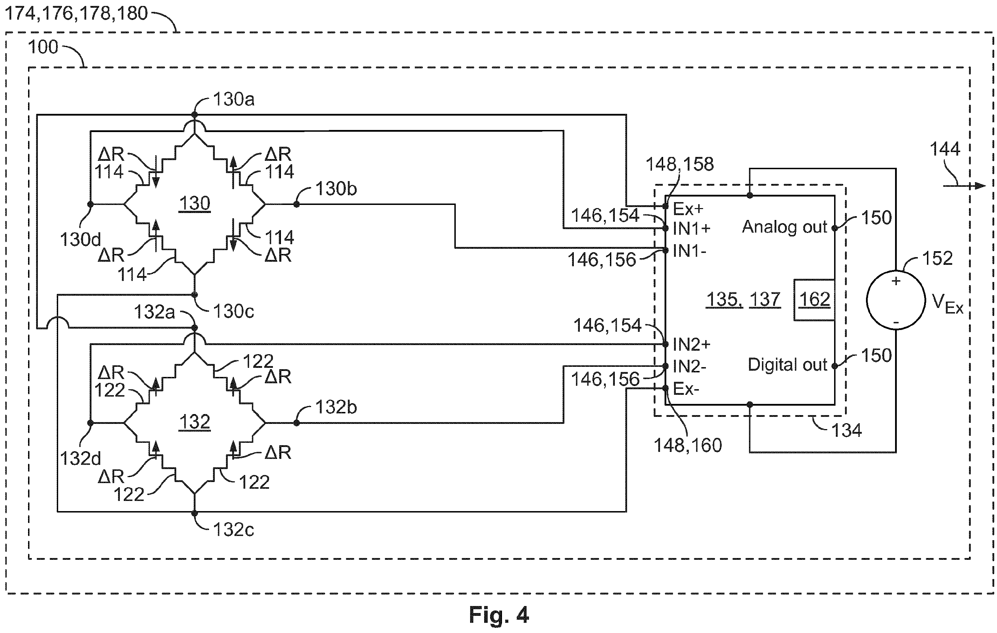

Figs. 4 to 7 show examples of configurations of piezoresistive sensor elements 100 with their processing circuits.

Moreover, in the examples shown in these Figures, the piezoresistive sensor element 100 is part of a sterilizable medical device 174, a sterile medical device 176, an invasive medical device 178, or an interface 180 of the sterile medical device 176.

Fig. 4 illustrates an example of a sensor element 100, which integrally comprises the first Wheatstone bridge 130 with the sensitive piezoresistors 114, the second Wheatstone bridge 132 with the in-sensitive piezoresistors 122, and an integrated signal processing unit 134. The integrated signal processing unit 134 is operable to calculate a drift-compensated sensor output signal 144. In this example, the signal processing unit 134 is or comprises an ASIC 135, and evaluates the first and second bridge signals separately.

Specifically, in Fig 4, each of the two Wheatstone bridges 130, 132 comprises four vertices indicated, in a clockwise order, by the letters a, b, c, and d. The references 130a and 132 a indicate, for instance, the vertex 'a' in the Wheatstone bridges 130, 132 respectively. Each pair of vertices are connected by a sensitive piezoresistors 114 or in-sensitive piezoresistors 122. Fig. 4 depicts the change in resistance, i.e. the resistance change $\Delta R$ due a mechanical stress on each piezoresistors. While the effect of mechanical stress is the same on both bridges 130, 132, the magnitude of the change on the piezoresistors of the second Wheatstone bridge 132 is significantly lower (ideally 0). The $\Delta R$ of all piezoresistors is due to a combination of mechanical, thermal and radiation effects.

The signal processing unit 134 may be connected to an external voltage source 152, which provides a voltage $V_{Ex}$, and have input terminals 146, output terminals 148, and reading terminals 150 that generate the sensor output signal 144 in the form of analog and/or digital signals.

The input terminals 146 comprise positive input terminals 154 and negative input terminals 156 which are connected to the two Wheatstone bridges 130, 132. In particular, in this embodiment, the vertex 130d is connected to a first positive input terminal 154, the vertex 130b is connected to a first negative input terminal 156, the vertex 132d is connected to a second positive input terminal 154, and the vertex 132b is connected to a second negative input terminal 156. Therefore, the signal processing unit 134 evaluates the voltage of each Wheatstone bridge 130, 132 separately.

The output terminals 148 comprise positive output terminals 158 and negative output terminals 160 which transfer the external voltage $V_{Ex}$ to the Wheatstone bridges 130, 132. The vertices 130a and 132a of the Wheatstone bridges 130, 132 are connected together to the positive output terminal 158 of the signal processing unit 134 and the vertices 130c and 132c are connected together to the negative output terminal 160 of the signal processing unit 134. Therefore, in this embodiment, the potential difference $V_{Ex}$ between the vertices 130a, 130c of the first Wheatstone bridge 130 is the same as between the vertices 132a, 132c of the second Wheatstone bridge 132.

All in all, in the embodiment of Fig. 4, the signal processing unit 134 has the following inputs: the voltage $V_{Ex}$ and the bridge signals, i.e. voltages, of the Wheatstone bridges 130, 132. From all these quantities, the signal processing unit 134 may compute the compensated output signal 144 of the piezoresistive sensor 100, for example after subtracting the second bridge signal from the first bridge signal. By doing so, the signal processing unit 134 compensates for contributions to the first and second bridge signals that are not due to mechanical stress, e.g. a long-term drift and/or an initial drift due to ionizing radiation.

Furthermore, the piezoresistive sensor element 100 may comprise a status indicator 162 for flagging the sensor condition. The status indicator 162 may be integrated in the signal processing unit 134 or in the ASIC 135 and be configured to provide a first flag output 164 before sterilization of the piezoresistive sensor element 100 and a second flag output 166 after sterilization of the piezoresistive sensor element 100 with a predetermined medically sterilizing dose.

The status indicator 162 may be configured to provide the second flag output 166 if a deviation of the first bridge signal from the second bridge signal exceeds a predetermined medically sterilizing signal threshold and to provide the first flag output 164 if a deviation of the first bridge signal from the second bridge signal does not exceed the predetermined medically sterilizing signal threshold. The predetermined medically sterilizing signal threshold is representative of the predetermined medically sterilizing dose.

The status indicator 162 may further be configured to provide a third flag output 168 when an ionizing radiation dose received by the piezoresistive sensor element 100 exceeds a predetermined damage dose beyond which the electronic circuity ceases to be usable. The status indicator 162 is configured to provide the third flag output 168 when the deviation of the first bridge signal from the second bridge signal exceeds a predetermined damage signal threshold that is representative of the predetermined damage dose.

The status indicator 162 may be accessible from outside and comprise a terminal or generate a digital signal.

Additionally, two temperature sensors (in particular, temperature diodes) 136 may be provided for sensing the temperature of the sensor element 100, so that further compensation calculations can be performed, thus rendering the output signal even more accurate.

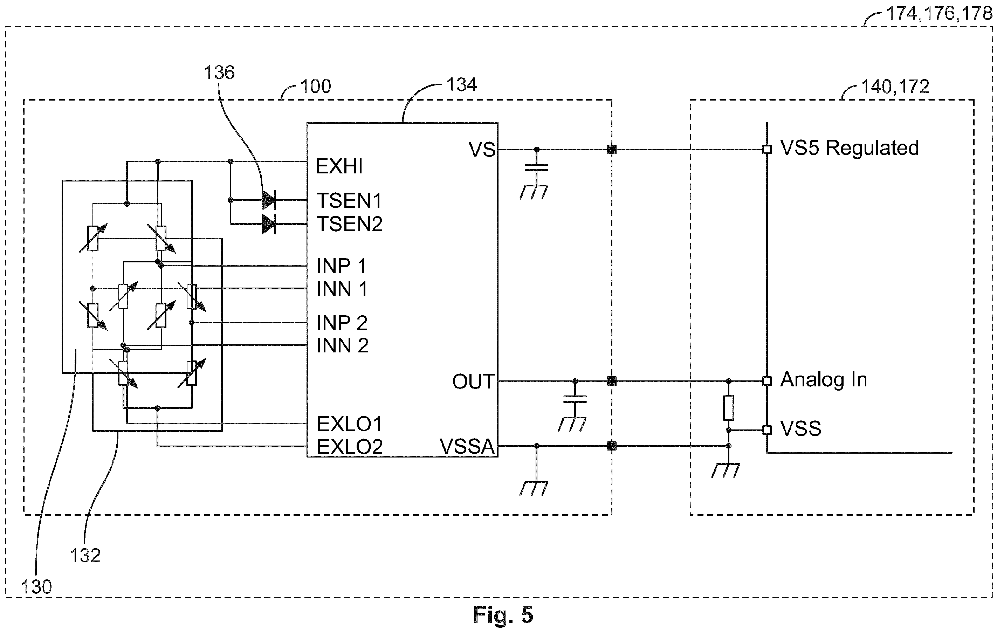

As shown in Fig. 5, all components may for instance be integrated together with an electronic control circuit (ECU) 140 or microcontroller 172.

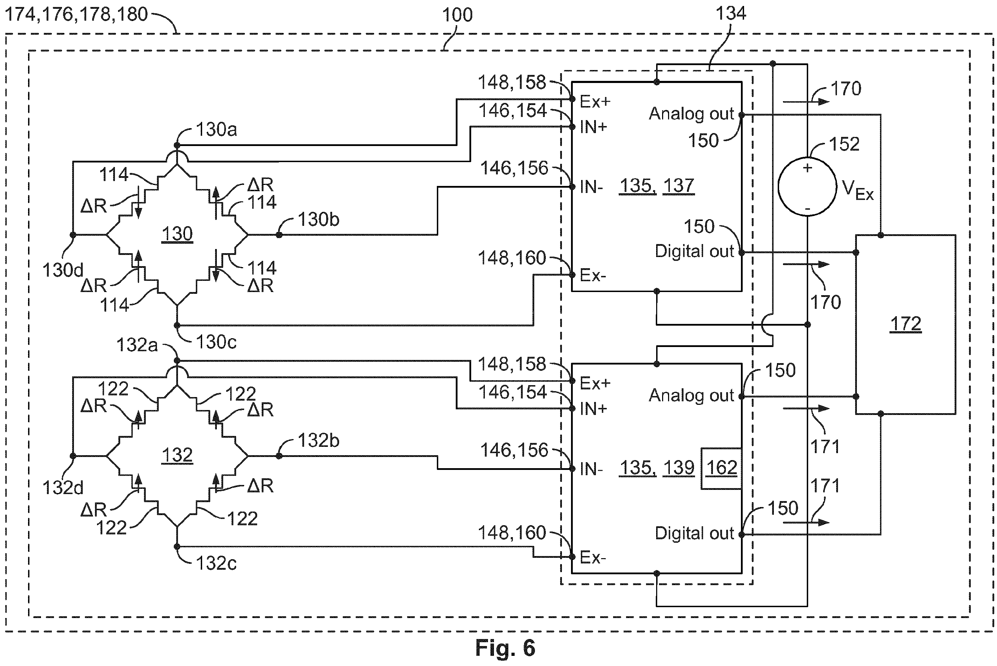

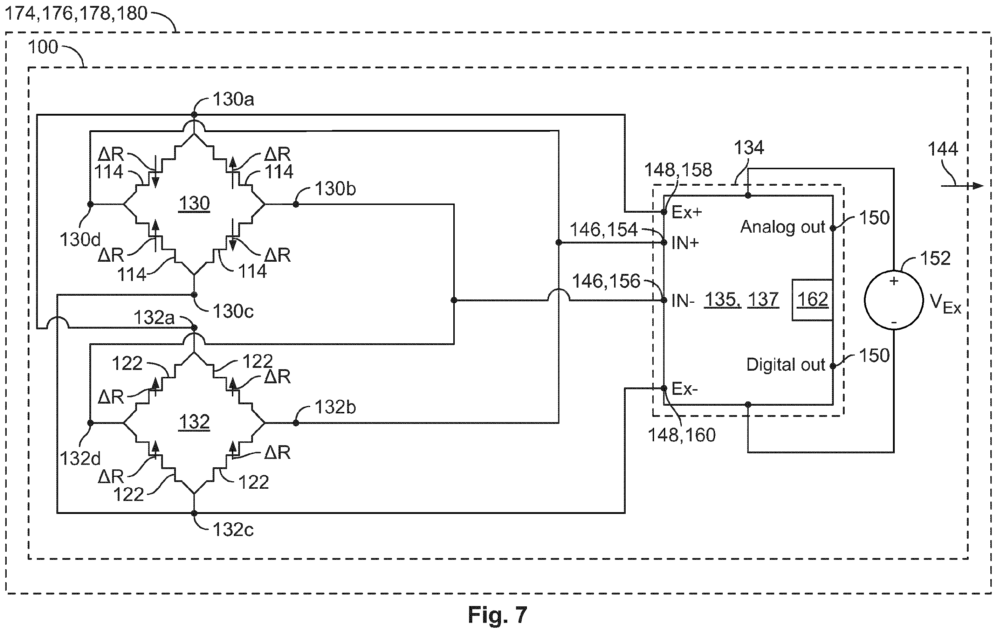

Figs. 6 and 7 show further alternatives to the embodiment of Figs. 4 and 5. These alternative embodiments differ from the previous embodiment in that the two Wheatstone bridges are made either more independent from each other (Fig. 6) or more dependent from each other (Fig. 7).

In Fig. 6, the two Wheatstone bridges are made more independent from each other: the signal processing unit 134 comprises two separate signal processing subunits 137, 139, each of which is or comprises an ASIC 135 and is connected to the same external voltage source 152 providing a voltage $V_{Ex}$. The signal processing subunits 137, 139 have input terminals 146, output terminals 148, and reading terminals 150 that generate preliminary signals 170, 171 in the form of analog and/or digital signals.

For example, the two input terminals 146 of the first signal processing subunit 137 may be connected to the vertices 130d and 130b of the first Wheatstone bridge 130, and the two input terminals 146 of the second ASIC 135 are connected to the vertices 132d and 132b of the second Wheatstone bridge 132.

Similarly, the output terminals 148 of the first signal processing subunit 137 are connected to the vertices 130a and 130c of the first Wheatstone bridge 130, and the output terminals 148 of the second signal processing subunit 139 are connected to the vertices 132a and 132c of the second Wheatstone bridge 132.

All in all, in the embodiment of Fig. 6, each of the two signal processing subunits 137, 139 has the following inputs: the voltage $V_{Ex}$ and the bridge signal, i.e. voltage, of the respective Wheatstone bridge 130, 132. The output comprises two preliminary signals 170, 171. In this configuration, a further microcontroller 172 may be needed to compute the difference between the first preliminary signal 170 and second preliminary signal 171 and thus compensate for nonmechanical effects like a long-term drift and/or an initial drift due to ionizing radiation. The second signal processing subunit 139 may also comprise a status indicator 162 functioning as described above.

Yet another alternative is considered in Fig. 7, wherein the two Wheatstone bridges 130, 132, contrarily to the embodiment of Fig. 6, are made strongly dependent from each other by cross-coupling: the configuration is the same as the one of Fig. 4, except for the following: the vertex 130d of the first Wheatstone bridge 130 is connected to the vertex 132b of the second Wheatstone bridge 132, and both are connected to the positive input terminal 154 of the signal processing unit 134; similarly, the vertex 130b of the first Wheatstone bridge 130 is connected to the vertex 132d of the second Wheatstone bridge 132, and both are connected to the negative input terminal 156 of the signal processing unit 134.

As a consequence, the signal processing unit 134 has the following inputs in this case: the voltage $V_{Ex}$ and one single signal, i.e. voltage, coming from the cross-coupling of the two Wheatstone bridges 130, 132. The subtraction between the first and second bridge signals, in this case, is performed physically and automatically by the cross-coupling of the two Wheatstone bridges 130, 132. This configuration has the disadvantage of reducing the sensitivity of the piezoresistive sensor element 100, whereas the configurations of Figs. 4, 5, and 6 do not have the same disadvantage, since the sensitivity remains unaffected.

In both the embodiments of Fig. 6 and 7, temperature sensors 136 may be provided as in the embodiments of Fig. 4 and 5.

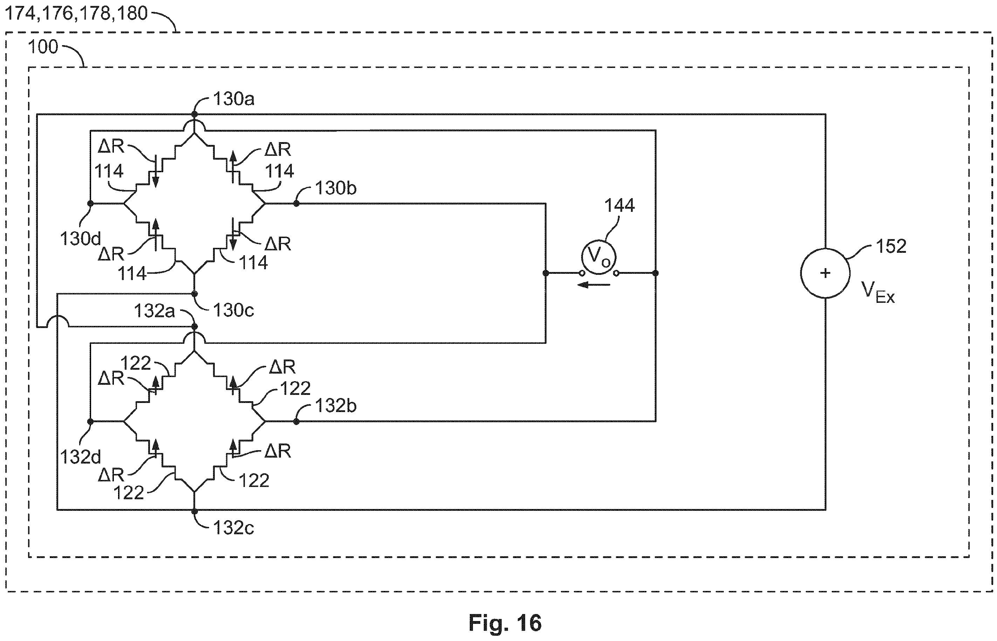

Fig. 16 shows a schematic concept of cross-coupling without an ASIC. The second Wheatstone bridge 132 that is insensitive to pressure is used to mirror the effect of sterilization. The second bridge 132 is sensitive to temperature and the magnitude of the effect of sterilization and temperature is much smaller than that of pressure. It varies in amount depending on the geometry and electrical conditions of each resistor but is typically in the same direction for all, hence the smaller arrows pointing in the same direction. Cross-coupling the output of the two bridges as shown will cancel the initial offset caused by sterilization of a calibrated sensor 100, or other drift, e.g. by temperature.

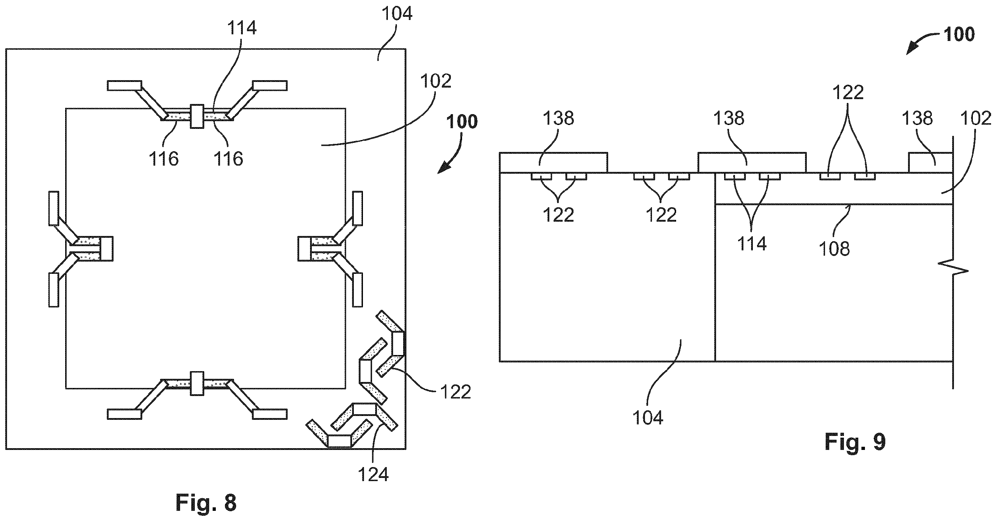

According to the example shown in Fig. 1 and 2, each of the in-sensitive piezoresistors 122 is placed in close vicinity to a corresponding sensitive piezoresistor 114. However, this does not necessarily have to be the case. The in-sensitive piezoresistors 122 may alternatively be located distanced away from the sensitive piezoresistors 114. An example of such an arrangement is depicted in Fig. 8. Here, the in-sensitive piezoresistors 122 are arranged in a peripheral region of the frame 104. Again, the resistive regions 124 of the stress in-sensitive piezoresistors 122 are laid out at an angle of 45 degrees compared to the resistive regions 116 of the stress sensitive piezoresistors 114. Thus, the stress sensitivity of in-sensitive piezoresistors 122 is zero or negligible compared to the stress sensitivity of the sensitive piezoresistors 114.

It has been found that the long-term drift effects that are to be tackled according to the present disclosure are partly screened by the usually applied passivation layers. The passivation for instance comprises a stack of a layer of silicon dioxide followed by a layer of silicon nitride. This finding is taken into account for the exemplary arrangement shown in Fig. 9.

According to Fig. 9, the passivation layer 138 is partly removed to augment the impact of the drift. In particular, the passivation layer 138 is removed from the regions where the in-sensitive piezoresistors 122 are located to facilitate the effects of the drift.

Furthermore, the backside 108 of the membrane 102 can also be a cause of drift due to electrical charges or due to leakage current, if the depth of the piezoresistors is close to the thickness of the membrane 102. As shown in Fig. 9, the in-sensitive piezoresistors 122 can be arranged on the frame 104 to make them as in-sensitive to stress as possible or on the membrane 102 to imitate the situation of the stress sensitive piezoresistors 114.

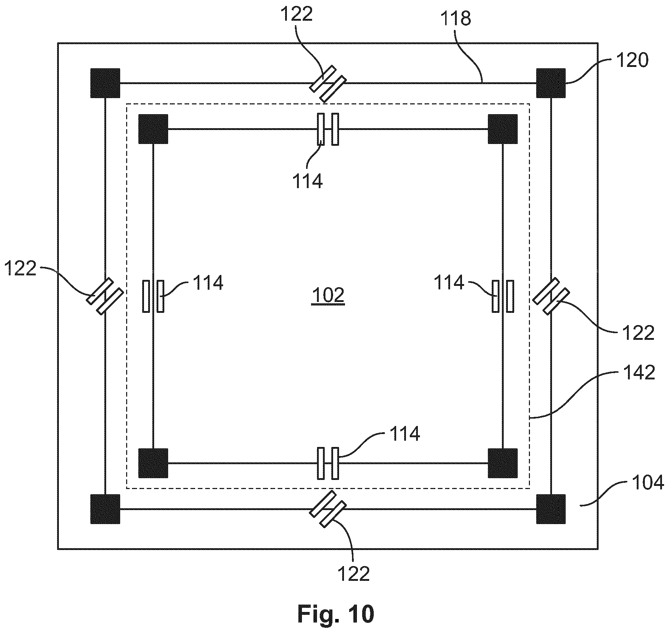

In Fig. 9, reference numeral 122 indicates stress insensitive resistors, whereas reference numeral 114 designates the stress sensitive piezoresistors. Another possibility would be to have the resistors 122 with a passivation on the membrane (i. e. the same arrangement as for the resistors 114, but in a stress insensitive orientation), but that arrangement would be less advantageous. Fig. 10 illustrates still another advantageous example of a piezoresistive pressure sensor 100. The periphery of the membrane is marked here by a broken line 142. Again, the resistive regions of the stress in-sensitive piezoresistors 122 are laid out at an angle of 45 degrees compared to the resistive regions of the stress sensitive piezoresistors 114. Thus, the stress sensitivity of insensitive piezoresistors 122 is zero or negligible compared to the stress sensitivity of the sensitive piezoresistors 114.

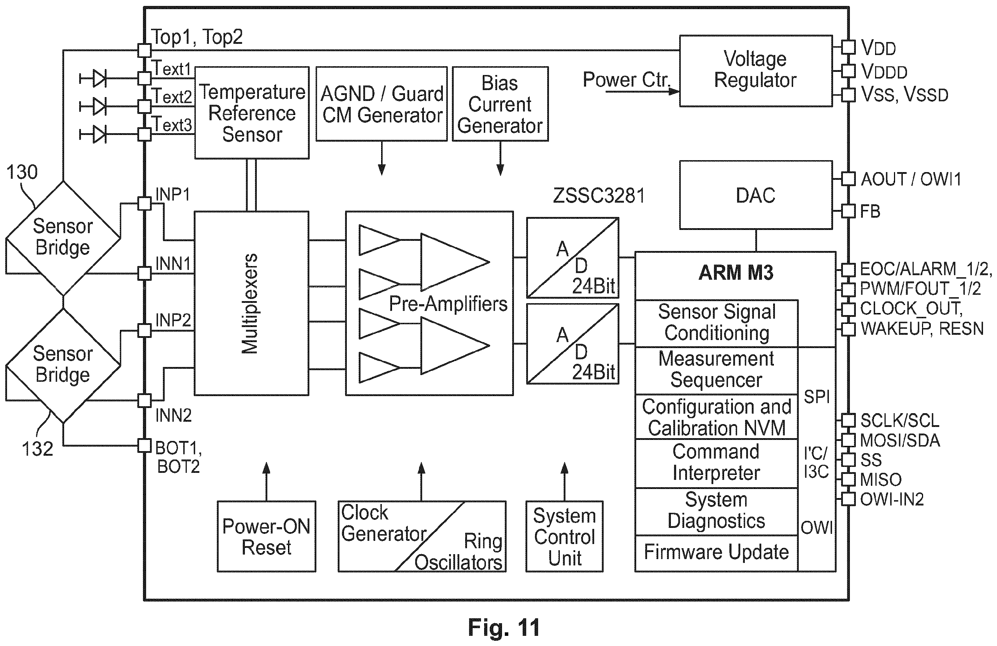

Fig. 11 illustrates a further example of an electronic circuit that can used for calculating the compensated sensor output signal from the output signals of the first Wheatstone bridge 130 with the sensitive piezoresistors and the second Wheatstone bridge 132 with the in-sensitive piezoresistors 122.

A compensation using an ASIC such as the dual channel 24-Bit resistive sensor signal conditioner with analog and digital output ZSSC3281 sold by the company Renesas Electronics is a mathematical operation using the output of the stress sensitive bridge and stress insensitive bridge. An example of generating the sensor output signal 144 based on a difference of the bridge signals is a subtraction of the stress insensitive output (i. e. the drift) from the stress sensitive output. Another example is to use the output of the stress insensitive bridge to trigger a warning when it exceeds a certain amount of drift. Another example, as required by claim 1, is to have the compensation based on the signal difference being performed directly by the two Wheatstone bridges 130, 132 if their outputs are physically connected, that is, if the two Wheatstone bridges 130, 132 are cross-coupled.

## REFERENCE NUMERALS

| Reference Numeral | Description |
|---|---|
| 100 | piezoresistive sensor element; pressure sensor |
| 102 | membrane, diaphragm |
| 104 | frame |
| 106 | void |
| 108 | first side of membrane, oriented towards void |
| 110 | second side of membrane, oriented towards outside of sensor |
| 112 | area with maximum stress |
| 114 | sensitive piezoresistor |
| 116 | ion implanted resistive region |
| 118 | connecting lead |
| 120 | contact pad |
| 122 | in-sensitive piezoresistor |
| 124 | ion implanted resistive region |
| 126 | diffused conductor |
| 128 | deposited metal layer |
| 130 | first Wheatstone bridge |
| 130a, 130b, 130c, 130d | vertices of the first Wheatstone bridges |
| 132 | second Wheatstone bridge |
| 132a, 132b, 132c, 132d | vertices of the second Wheatstone bridges |
| 134 | signal processing unit |
| 135 | application-specific integrated circuit (ASIC) |
| 136 | temperature sensor |
| 137 | first signal processing subunit |
| 138 | passivation |
| 139 | second signal processing subunit |

(continued)

| Reference Numeral | Description |
|---|---|
| 140 | ECU |
| 142 | periphery of membrane |
| 144 | sensor output signal |
| 146 | input terminal |
| 148 | output terminal |
| 150 | reading terminal |
| 152 | voltage source |
| 154 | positive input terminal |
| 156 | negative input terminal |
| 158 | positive output terminal |
| 160 | negative output terminal |
| 162 | status indicator |
| 164 | first flag output |
| 166 | second flag output |
| 168 | third flag output |
| 170 | first preliminary signal |
| 171 | second preliminary signal |
| 172 | microcontroller |
| 174 | sterilizable medical device |
| 176 | sterile medical device |
| 178 | invasive medical device |
| 200 | conventional piezoresistive pressure sensor |
| 202 | diaphragm |
| 204 | frame |
| $\Delta R$ | resistance change |

**Claims**

1. Piezoresistive sensor element (100) comprising:

   a substrate, which in operation is subjected to mechanical stress in response to a measurand to be measured, a first array of at least four sensitive piezoresistors (114), wherein the sensitive piezoresistors (114) are arranged on the substrate and are connected to form a first Wheatstone bridge for generating a first bridge signal, a second array of at least four in-sensitive piezoresistors (122), wherein the in-sensitive piezoresistors (122) are connected to form a second Wheatstone bridge for generating a second bridge signal, wherein the sensitive piezoresistors (114) have a stress sensitivity which is higher than the stress sensitivity of the in-sensitive piezoresistors (122), and wherein an output signal of the piezoresistive sensor (100) is generated based on a difference of the first and second bridge signals, **characterized in that** the in-sensitive piezoresistors (122) are arranged to include an angle of 45 degrees with each of the sensitive piezoresistors (114), and wherein the first Wheatstone bridge (130) is cross-coupled to the second Wheatstone bridge (132).

2. Piezoresistive sensor element (100) according to claim 1, wherein each of the in-sensitive piezoresistors (122) is arranged in close proximity to one corresponding sensitive piezoresistor (114), so as to be subjected to essentially the same stress as the corresponding sensitive piezoresistor (114).

3. Piezoresistive sensor element (100) according to any of claims 1 to 2, further comprising a signal processing unit (134) for evaluating the first bridge signal and the second bridge signal and for generating the sensor output signal (144).

4. Piezoresistive sensor element (100) according to claim 3, wherein the signal processing unit (134) comprises a first signal processing subunit (137) for evaluating the first bridge signal and generating a first preliminary signal (170) and a second signal processing subunit (139) for evaluating the second bridge signal and generating a second preliminary signal (171).

5. Piezoresistive sensor element (100) according to claim 4, further comprising a microcontroller (172) connected to the signal processing subunits (137, 139) for computing the sensor output signal (144) based on a difference between the first preliminary signal (170) and the second preliminary signal (171).

6. Piezoresistive sensor element (100) according to any of claims 1 to 5, further comprising a status indicator (162) configured to provide a first flag output (164) before sterilization of the piezoresistive sensor element (100) and a second flag output (166) after sterilization of the piezoresistive sensor element (100).

7. Piezoresistive sensor element (100) according to claim 6, wherein the status indicator (162) is configured to provide the second flag output (166) if a deviation of the first bridge signal from the second bridge signal exceeds a predetermined medically sterilizing signal threshold.

8. Piezoresistive sensor element (100) according to claim 6 or 7, wherein the status indicator (162) is further configured to provide a third flag output (168) when an ionizing radiation dose received by the piezoresistive sensor element (100) exceeds a predetermined damage dose threshold.

9. Sterilizable medical device (174) for pressure measurements comprising a piezoresistive sensor element (100) according to any of the preceding claims and being configured to be sterilized by ionizing radiation and/or ethylene oxide.

10. Sterile medical device (176) for pressure measurements comprising the sterilizable medical device (174) of claim 9, wherein the sterilizable medical device (174) has been sterilized, preferably by ionizing radiation or by ethylene oxide.

11. Sterile medical device (176) for pressure measurements according to claim 10, comprising an interface (180) configured for sterile coupling to a patient.

12. Sterile medical device (176) for pressure measurements according to claim 10 or 11, configured to be in contact with a body fluid of the patient.

13. Invasive medical device (178), configured to be sterilized by ionizing radiation and/or ethylene oxide and comprising the piezoresistive pressure sensor (100) of any of claims 1 to 8, the invasive medical device (178) being configured to contact a body fluid of a patient, the piezoresistive pressure sensor (100) being configured to measure a pressure of said body fluid.

14. Use of the piezoresistive sensor element (100) according to any one of claims 1 to 8 in a sterilizable medical device (174) as defined in claim 9.

**Patentansprüche**

1. Piezoresistives Sensorelement (100), mit:

   einem Substrat, das im Betrieb als Reaktion auf eine zu messende Größe einer mechanischen Belastung ausgesetzt ist,
   einer ersten Anordnung von mindestens vier empfindlichen Piezowiderständen (114), wobei die empfindlichen Piezowiderstände (114) auf dem Substrat angeordnet und so verbunden sind, dass sie eine erste Wheatstone-Brücke zur Erzeugung eines ersten Brückensignals bilden,

einer zweiten Anordnung von mindestens vier unempfindlichen Piezowiderständen (122), wobei die unempfindlichen Piezowiderstände (122) so verbunden sind, dass sie eine zweite Wheatstone-Brücke zur Erzeugung eines zweiten Brückensignals bilden,

wobei die empfindlichen Piezowiderstände (114) eine Belastungsempfindlichkeit aufweisen, die höher ist als die Belastungsempfindlichkeit der unempfindlichen Piezowiderstände (122), und wobei ein Ausgangssignal des piezoresistiven Sensors (100) auf der Grundlage einer Differenz des ersten und des zweiten Brückensignals erzeugt wird,

**dadurch gekennzeichnet, dass**

die unempfindlichen Piezowiderstände (122) so angeordnet sind, dass sie mit jedem der empfindlichen Piezowiderstände (114) einen Winkel von 45 Grad einschließen, und

die erste Wheatstone-Brücke (130) mit der zweiten Wheatstone-Brücke (132) gekoppelt ist.

2. Piezoresistives Sensorelement (100) nach Anspruch 1, wobei jeder der unempfindlichen Piezowiederstanden (122) in unmittelbarer Nähe zu einem entsprechenden empfindlichen Piezowiederstand (114) angeordnet ist derart, dass er im Wesentlichen derselben Belastung ausgesetzt ist wie der entsprechende empfindliche Piezowiederstand (114).

3. Piezoresistives Sensorelement (100) nach einem der Ansprüche 1 bis 2, das ferner eine Signalverarbeitungseinheit (134) zum Auswerten des ersten Brückensignals und des zweiten Brückensignals sowie zum Erzeugen des Sensorausgangssignals (144) aufweist.

4. Piezoresistives Sensorelement (100) nach Anspruch 3, wobei die Signalverarbeitungseinheit (134) eine erste Signalverarbeitungsteileinheit (137) zur Auswertung des ersten Brückensignals und zur Erzeugung eines ersten Vorab-Signals (170) sowie eine zweite Signalverarbeitungsteileinheit (139) zur Auswertung des zweiten Brückensignals und zur Erzeugung eines zweiten Vorab-Signals (171) aufweist.

5. Piezoresistives Sensorelement (100) nach Anspruch 4, das ferner einen mit den Signalverarbeitungsteileinheiten (137, 139) verbundenen Mikrocontroller (172) aufweist, der das Sensorausgangssignal (144) auf der Grundlage einer Differenz zwischen dem ersten Vorab-Signal (170) und dem zweiten Vorab-Signal (171) berechnet.

6. Piezoresistives Sensorelement (100) nach einem der Ansprüche 1 bis 5, das ferner einen Statusindikator (162) umfasst, der ausgebildet ist, vor einer Sterilisation des piezoresistiven Sensorelements (100) ein erstes Markierungsausgangssignal (164) und nach der Sterilisation des piezoresistiven Sensorelements (100) ein zweites Markierungsausgangssignal (166) bereitzustellen.

7. Piezoresistives Sensorelement (100) nach Anspruch 6, wobei der Statusindikator (162) ausgebildet ist, das zweite Markierungsausgangssignal (166) bereitzustellen, wenn eine Abweichung des ersten Brückensignals von dem zweiten Brückensignal einen vorbestimmten Schwellenwert für medizinische Sterilisierungssignale überschreitet.

8. Piezoresistives Sensorelement (100) nach Anspruch 6 oder 7, wobei der Statusindikator (162) ferner ausgebildet ist, ein drittes Markierungsausgangssignal (168) bereitzustellen, wenn eine von dem piezoresistiven Sensorelement (100) aufgenommene ionisierende Strahlungsdosis einen vorbestimmten Schwellenwert bezüglich einer schädigenden Dosis überschreitet.

9. Sterilisierbare medizinische Vorrichtung (174) zur Druckmessung, die ein piezoresistives Sensorelement (100) nach einem der vorstehenden Ansprüche aufweist und ausgebildet ist, durch ionisierende Strahlung und/oder Ethylenoxid sterilisierbar zu sein.

10. Sterile medizinische Vorrichtung (176) zur Druckmessung mit der sterilisierbaren medizinischen Vorrichtung (174) nach Anspruch 9, wobei die sterilisierbare medizinische Vorrichtung (174) vorzugsweise durch ionisierende Strahlung oder durch Ethylenoxid sterilisiert ist.

11. Sterile medizinische Vorrichtung (176) zur Druckmessung nach Anspruch 10, mit einer Kopplungsstelle (180), die für eine sterile Kopplung an einen Patienten ausgelegt ist.

12. Sterile medizinische Vorrichtung (176) zur Druckmessung nach Anspruch 10 oder 11, die ausgelegt ist, mit einer Körperflüssigkeit des Patienten in Kontakt zu stehen.

13. Invasive medizinische Vorrichtung (178), die ausgelegt ist, durch ionisierende Strahlung und/oder Ethylenoxid sterilisierbar zu sein, und die das piezoresistive Sensorelement (100) nach einem der Ansprüche 1 bis 8 aufweist, wobei die invasive medizinische Vorrichtung (178) ausgelegt ist, mit einer Körperflüssigkeit eines Patienten in Kontakt zu kommen, und das piezoresistive Sensorelement (100) ausgelegt ist, einen Druck der Körperflüssigkeit zu messen.

14. Verwendung des piezoresistiven Sensorelements (100) nach einem der Ansprüche 1 bis 8 in einer in Anspruch 9 definierten sterilisierbaren medizinischen Vorrichtung (174).

**Revendications**

1. Élément de capteur piézorésistif (100) comprenant :

un substrat qui, en fonctionnement, est soumis à une contrainte mécanique en réponse à un mesurande à mesurer,
un premier réseau d'au moins quatre piézorésistances sensibles (114), dans lequel les piézorésistances sensibles (114) sont agencées sur le substrat et sont connectées pour former un premier pont de Wheatstone permettant de générer un premier signal de pont,
un deuxième réseau d'au moins quatre piézorésistances in-sensibles (122), dans lequel les piézorésistances in-sensibles (122) sont connectées pour former un deuxième pont de Wheatstone pour générer un deuxième signal de pont,
dans lequel les piézorésistances sensibles (114) ont une sensibilité à la contrainte qui est supérieure à la sensibilité à la contrainte des piézorésistances in-sensibles (122), et dans lequel un signal de sortie du capteur piézorésistif (100) est généré sur la base d'une différence entre le premier et le deuxième signal de pont, **caractérisé en ce que** les piézorésistances in-sensibles (122) sont agencées pour inclure un angle de 45 degrés avec chacune des piézorésistances sensibles (114), et
dans lequel le premier pont de Wheatstone (130) est couplé en croix au deuxième pont de Wheatstone (132).

2. Élément capteur piézorésistif (100) selon la revendication 1, dans lequel chacune des piézorésistances in-sensibles (122) est agencée à proximité d'une piézorésistance sensible (114) correspondante, de manière à être soumise essentiellement à la même contrainte que la piézorésistance sensible (114) correspondante.

3. Élément de capteur piézorésistif (100) selon l'une quelconque des revendications 1 à 2, comprenant en outre une unité de traitement de signal (134) pour évaluer le premier signal de pont et le deuxième signal de pont et pour générer le signal de sortie du capteur (144).

4. Élément de capteur piézorésistif (100) selon la revendication 3, dans lequel l'unité de traitement de signal (134) comprend une première sous-unité de traitement de signal (137) pour évaluer le premier signal de pont et générer un premier signal préliminaire (170) et une deuxième sous-unité de traitement de signal (139) pour évaluer le deuxième signal de pont et générer un deuxième signal préliminaire (171).

5. Élément de capteur piézorésistif (100) selon la revendication 4, comprenant en outre un microcontrôleur (172) connecté aux sous-unités de traitement de signal (137, 139) pour calculer le signal de sortie du capteur (144) sur la base d'une différence entre le premier signal préliminaire (170) et le deuxième signal préliminaire (171).

6. Élément de capteur piézorésistif (100) selon l'une quelconque des revendications 1 à 5, comprenant en outre un indicateur d'état (162) configuré pour fournir un premier indicateur de sortie (164) avant la stérilisation de l'élément de capteur piézorésistif (100) et un deuxième indicateur de sortie (166) après la stérilisation de l'élément de capteur piézorésistif (100).

7. Élément de capteur piézorésistif (100) selon la revendication 6, dans lequel l'indicateur d'état (162) est configuré pour prévoir le deuxième indicateur de sortie (166) si un écart entre le premier signal de pont et le deuxième signal de pont dépasse un seuil de signal de stérilisation médicale prédéterminé.

8. Élément de capteur piézorésistif (100) selon la revendication 6 ou 7, dans lequel l'indicateur d'état (162) est en outre configuré pour fournir un troisième indicateur de sortie (168) lorsqu'une dose de rayonnement ionisant reçue par l'élément de capteur piézorésistif (100) dépasse un seuil de dose de dégâts prédéterminé.

9. Dispositif médical stérile (174) de mesure de pression comprenant un élément de capteur piézorésistif (100) selon l'une quelconque des revendications précédentes et étant configuré pour être stérilisé par rayonnement ionisant et/ou par oxyde d'éthylène.

10. Dispositif médical stérile (176) de mesure de pression comprenant le dispositif médical stérile (174) selon la revendication 9, dans lequel le dispositif médical stérile (174) a été stérilisé, de préférence par rayonnement ionisant ou par oxyde d'éthylène.

11. Dispositif médical stérile (176) de mesures de pression selon la revendication 10, comprenant une interface (180) configurée pour être couplée stérilement à un patient.

12. Dispositif médical stérile (176) de mesure de pression selon la revendication 10 ou 11, configuré pour être en contact avec un fluide corporel du patient.

13. Dispositif médical invasif (178), configuré pour être stérilisé par rayonnement ionisant et/ou oxyde d'éthylène et comprenant le capteur de pression piézorésistif (100) selon l'une quelconque des revendications 1 à 8, le dispositif médical invasif (178) étant configuré pour être en contact avec un fluide corporel d'un patient, le capteur de pression piézorésistif (100) étant configuré pour mesurer une pression dudit fluide corporel.

14. Utilisation de l'élément de capteur piézorésistif (100) selon l'une quelconque des revendications 1 à 8 dans un dispositif médical stérile (174) tel que défini selon la revendication 9.

**Fig. 1**

**Fig. 2**

EP 4 567 394 B1

Fig. 3

Fig. 4

Fig. 5

Fig. 6

**Fig. 7**

EP 4 567 394 B1

Fig. 8

Fig. 9

EP 4 567 394 B1

Fig. 10

EP 4 567 394 B1

Fig. 11

Fig. 13

Fig. 12

**Fig. 14**

**Fig. 15**

EP 4 567 394 B1

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3287758 B1
- EP 22198016

- US 7856885 B1

**Non-patent literature cited in the description**

- **BARLIAN, A. ALVIN** ; **PARK, WOO-TAE** ; **MALLON, JOSEPH R., JR.** ; **RASTEGAR, ALI J.** ; **PRUITT, BETH L.** Review: Semiconductor Piezoresistance for Microsystems. *Proceedings of the IEEE. Institute of Electrical and Electronics Engineers*, 2009, vol. 97, 513-552

- **SMITH, C.S.** Piezoresistance Effect in Germanium and Silicon.. *Physical Review*, 1954, vol. 94, 42-49